# EUROPEAN PATENT APPLICATION

(11) **EP 2 708 290 A2**
(43) Date of publication of application: **19.03.2014**
(21) Application number: 13184684.2
(22) Date of filing: 17.09.2013
(51) Int. Cl.: B05B 12/14, B05B 15/12, B05B 12/04, A61M 35/00

(54) **System and method for automatically controlling application of skin treatment solution**

(30) Priority: 17.09.2012 US 201261702180 P; 17.09.2012 US 201261702194 P; 19.10.2012 US 201261716224 P
(71) Applicant: Sunless, Inc., Macedonia, OH 44056 (US)
(72) Inventor: Cooper, Steven, Macedonia, OH Ohio 44056 (US); Thomason, Scott, Macedonia, OH Ohio 44056 (US)
(74) Representative: Konkonen, Tomi-Matti Juhani

(57) **Abstract**

Embodiments disclosed herein propose controlling the application of a skin treatment solution spray using data obtained from a machine readable tag associated with a container filled with the skin treatment solution. A skin treatment sprayer system includes at least one container receptacle configured to receive a container of with skin treatment solution. An interrogator is coupled to the container receptacle and is operable to communicate with the machine readable tag associated with the container. A controller includes a processor and is in communication with the interrogator. At least one nozzle is operable to emit a spray of skin treatment solution and is in fluid communication with the container. The controller is operable to control a spray parameter associated with the emission of the skin treatment solution based on data received from the machine readable tag.

## Description

### PRIORITY CLAIM

This application claims priority to U.S. Provisional Patent Application No. 61/702,180 filed on September 17, 2012, and entitled System and Method for Automatically Controlling Application of Skin Treatment Solution, and to U.S. Provisional Patent Application No. 61/716,224 filed on October 19, 2012, and entitled System and Method for Accessing Remote Skin Treatment Solution Data, and to U.S. Provisional Patent Application No. 61/702,194 filed on September 17, 2012, and entitled System and Method for Accessing Remote Skin Treatment Solution Data, the disclosures of which are hereby incorporated by reference.

### TECHNICAL FIELD

The present invention relates generally to skin treatment sprayer systems, and more particularly to using information derived from a container to control parameters for the delivery of skin treatment solutions.

### BACKGROUND

Booth spray systems for the application of skin lotions and cosmetics dispense a selectable variety of skin treatments including moisturizer and tanning treatments. Salon spray booths for sunless tanning and skin treatments offer multiple spray sessions with selections from a wide variety of skin lotions and tanning products. Many of the booth systems have moving gantries that apply the spray evenly over the full body or can be user-programmed to apply only to the face or legs. Some booths are outfitted with booth pre-heaters and full body drying systems. Automated booth spray systems used in salons consist basically of a booth structure that is either fully or partially enclosed with single or multiple spray nozzles positioned inside the booth. Reference is made to the following references generally directed to booth-type spray systems, the disclosures of which are hereby incorporated by reference: U.S. Patent No. 6,199,557 to Laughlin filed on April 19, 1999; U.S. Patent No. 7,004,407 to Cooper filed on December 4, 2002; U.S. Patent No. 7,886,684 to Cooper et al. filed on April 28, 2006; U.S. Patent No. 8,201,288 to Thomason et al. filed on August 24, 2009; U.S. Patent Application Publication No. 2010/0266776 by Cooper et al. filed on April 20, 2010; and U.S. Patent Application Publication No. 2011/0133004 by Thomason et al. filed on October 22, 2010.

The spray session is activated by the person receiving the spray treatment within the enclosure. An exhaust fan may be used to prevent overspray inside the booth or drifting spray escaping from the booth. Other booth features may include lights, voice prompts in different languages, heaters, skin drying systems, and interior washing and rinsing systems. Recently, spray booths offer the user an option to select a level of tan, which corresponds to varying the volume of spray treatment dispensed base on the selected level of tan.

The electrical and mechanical components in the rinse, drain, spray, gantry, heat and exhaust systems of these automated spray booths are operated in a sequence during a spray session by a microprocessor based or other sequential controller with a manual input device such as a keypad or button panel. Spray session parameters such as liquid flow, duration of spray, heater temperature, and the like are set and adjusted by input to the controller. Salon personnel and/or consumers using the spray system may manually enter certain operation parameters for each spray session.

The spray solution used for sunless tanning is generally a water-based mixture of DHA (dihydroxyacetone) and/or erythrulose and various other skin care ingredients such as aloe vera. Often a cosmetic bronzer is added along with pleasant scents and other ingredients to enhance the tanning results and experience, such as formulations to balance skin pH. For best results, the spraying of the solution utilizes a finely atomized spray (mist), as opposed to using a spray stream or large spray droplets, because the mist of solution provides even coverage and reduces the risk of streaking or running of the spray deposit.

The spray systems of these booth-type skin treatment sprayers generally include single or multiple tanks containing liquid spray solution which is fed to the spray nozzles by single or multiple pumps or other methods, such as gravity or Venturi. Flow is generally controlled by solenoid valves and/or check valves and a mechanical pump, for which flow rate can be varied by motor speed or pressure. Multiple nozzles may be stationary, positioned along the interior walls of the booth, or they may be mounted to a moving gantry. A spray system disclosed in U.S. Patent No. 7,886,684 to Cooper et al., the disclosure of which is hereby incorporated by reference, utilizes a single dose cartridge tank system. This can be configured with a single nozzle that oscillates while moving on a gantry. In addition, it can be operated without a mechanical pump, relying on gravity or Venturi feed to the nozzle.

Multiple batch tank systems on skin care booth sprayers allow approximately 30 to more than 100 sessions between changing or re-filling the tanks. Booths with multiple tanks have the advantage of allowing a sequence of spray sessions with a choice of various lotions applied one after the other; for instance a moisturizer treatment may be applied after a tanning treatment, or a skin pH balancing spray may be applied before a tanning spray. Some booth models use refillable multiple tank systems with 2, 3 or 4 tanks. Many booth spray systems accommodate a more convenient bag-in-box system where multiple refillable and/or replaceable containers are received in a bay drawer of the unit as disclosed by U.S. Patent No. 8,201,288 to Thomason et al. filed on August 24, 2009, the disclosure of which is hereby incorporated by reference.

Sprayer systems operate based on a variety of parameters. For example, a sprayer system spraying one type of skin treatment solution may require a certain set of operational parameters to effectively deliver the solution, while delivery of a different skin treatment solution may be accomplished according to a different set of operational parameters. Manually configuring a sprayer system for different operational parameters for the delivery of different solutions is time consuming, often confusing, and prone to error.

### SUMMARY

Embodiments disclosed herein propose controlling the application of a skin treatment solution spray using data obtained from a machine readable tag associated with a container filled with the skin treatment solution. A skin treatment sprayer system includes at least one container receptacle configured to receive a container of skin treatment solution. An interrogator is coupled to the container receptacle and is operable to communicate with the machine readable tag associated with the container. A controller includes a processor and is in communication with the interrogator. At least one nozzle is operable to emit a spray of skin treatment solution and is in fluid communication with the container. The controller is operable to control a spray parameter associated with the emission of the skin treatment solution based on data received from the machine readable tag.

A method for delivering a skin treatment solution according to embodiments of the present disclosure includes receiving a container containing the skin treatment solution and having a machine readable tag coupled to the container. Data is received from the machine readable tag, and delivery of the skin treatment solution is controlled based on the received data.

In certain embodiments, the machine readable tag may be an RFID tag. In other embodiments, the machine readable tag may include a bar code or other information that may be optically or mechanically read or otherwise received by an interrogator. Controlling the delivery of the skin treatment solution may include controlling a pump rate, which directs a specific volume and/or a rate of delivery of the skin treatment solution.

Technical advantages of the system and method for controlling a skin treatment spray system include the ability to employ codes on a machine readable tag or a container including a code or other data that is configured to be read by a machine to ensure that only the proper solution is sprayed by the sprayer. Other technical advantages include the ability for a spray skin treatment system to receive purging and calibration data associated with a particular skin treatment solution and execute the purging and calibration operations automatically based on the data with little or no human involvement.

Still further technical advantages include the ability to maintain optimal performance of the spray system through efficient updates to spray parameters and other data used by the system through communication between the sprayer system and the container tag and/or communication between the sprayer system and a remote computing system, which may be part of a cloud computing network.

Other technical advantages will be readily apparent to one of ordinary skill in the art from the following figures, descriptions, and claims. Moreover, while specific advantages have been enumerated above, various embodiments may include all, some, or none of the enumerated advantages.

### BRIEF DESCRIPTION OF THE DRAWINGS

A more complete understanding of the invention may be obtained by reference to the following drawings:
Figures 1A and 1B schematically illustrate a container controlled spraying system adapted for spraying a skin treatment solution;
Figure 2A illustrates a flow diagram of a method of using solution specific data from a container tag to configure parameters of a spray system;
Figure 2B illustrates a flow diagram of using the data received from a container tag to control a spray session according to embodiments of the present disclosure;
Figure 3 illustrates an embodiment of a skin treatment spray booth;
Figure 4 is a detailed illustration of the receiver drawer shown in Figure 3;
Figure 5 is a portion of a container for containing a skin treatment solution with portions broken away to show a location of a container tag;
Figure 6 is a detailed illustration of the receiver drawer shown in Figure 3 and components supported thereby; and
Figure 7 is a table of data that may be coded to a container tag according to embodiments of the present disclosure.

### DETAILED DESCRIPTION

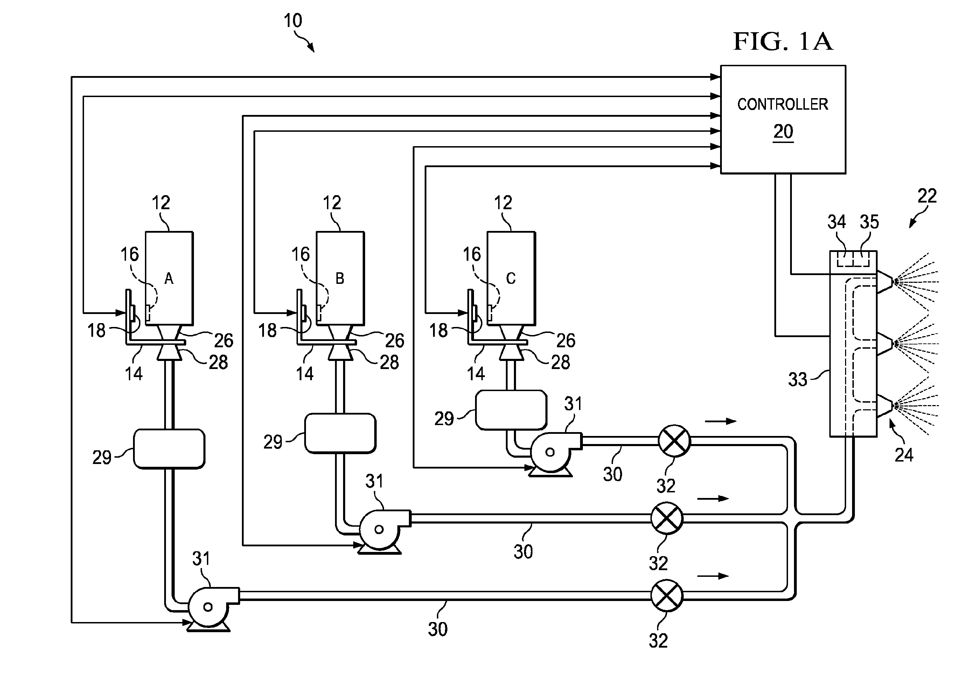

Reference is made to Figure 1A, which is a block diagram of a container controlled spray system 10 according to embodiments of the present disclosure. The container controlled spray system 10 employs machine readable tags associated with one or more containers to direct the control of various functions of the spray system, including controlling spray parameters. As described in more detail below, machine readable tags or tags as used herein denote any technique for storing and communicating data to a machine. For example, a machine readable tag may be optically read, such a 2-D or 3-D bar code; it may be magnetically read, such as a magnetic strip; it may be mechanically read, such as punched holes; or it may be electronically read, such as an RFID device.

The spray parameters are controlled such that a skin treatment solution is effectively sprayed and received on the skin of a user. Thus, data from the machine readable tags is read and operational parameters such as spray passes, drying passes, pump rate, gantry motor speed, and the like are determined based at least partially on the data received from the machine readable tags. In certain embodiments, the container controlled spray system 10 may be employed to spray sunless tanning solution on the skin of a user.

Referring to Figure 1A, a plurality of containers 12 are each installed in respective container receptacles, for example container bays 14. The container receptacles may be any suitable device for receiving a container of skin treatment solution. In one embodiment, the container receptacle may be a single application cartridge that is received in a receptacle coupled to a moving gantry that translates and oscillates a spray nozzle. Each container 12 includes a machine readable tag 16 that is fixed to, a part of, or otherwise associated with a respective container 12. The container tag 16 is encoded with data that the spray system 10 may use to perform a variety of different functions. The data may be specific to the contents of the particular container 12.

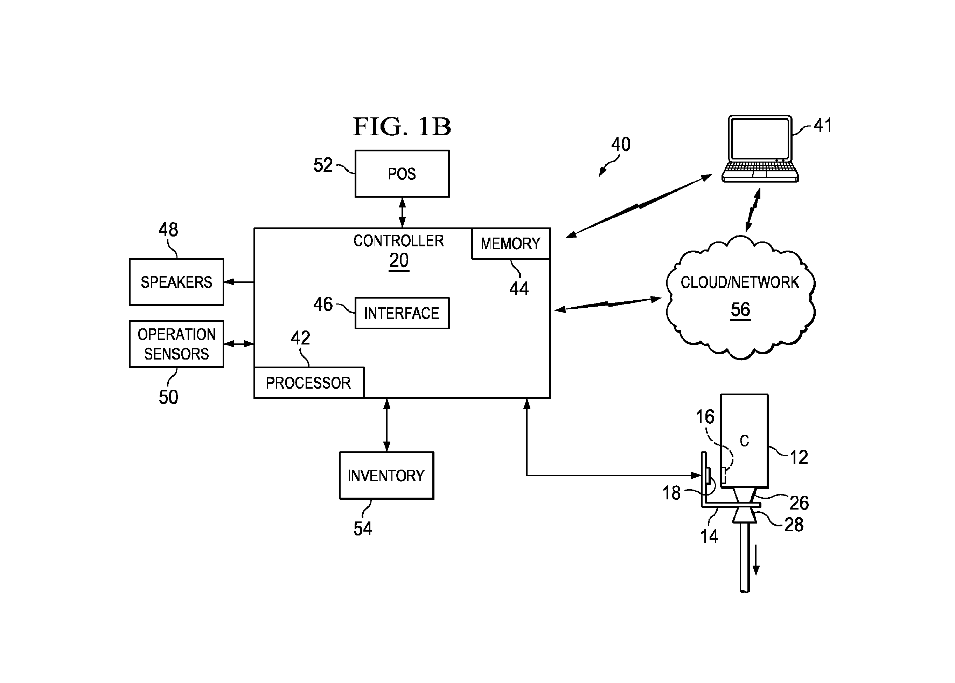

An interrogator 18 reads the data from the container tag 16 and communicates this data to the controller 20. The interrogator 18 may be coupled to the container receptacle as shown, or the interrogator 18 may be remote to the container receptacle. For example, the interrogator 18 may be associated with a computing device that is in wireless data communication with the controller 20. For example, the interrogator may be associated with a tablet, a smartphone, or a laptop or desktop computer. The controller 20 interprets the data obtained directly or indirectly from interrogation of the container tag 16 and communicates commands to the sprayer 22 to control the output of a nozzle 24 and other components associated with the sprayer 22 to ensure a pleasant and effective skin treatment solution spray experience. The controller 20 performs a variety of other functions including communicating the data to and receiving data from other systems that support a spray tanning operation as described further below with respect to Figure 1B.

The container 12 with the attached container tag 16 is received in a suitable receptacle, such as container bay 14 such that a fitting 26 of the container 12 is received by a corresponding receptacle fitting 28 of the container bay 14. The receptacle fitting 28 is fluidly coupled through a solution conduit 30 with the sprayer 22. Liquid flows from the receptacle fitting 28 through the solution conduit 30 and is received and emitted by the sprayer 22. The flow of solution through the respective solution conduits 30 may be controlled by a pump 31 and a conduit valve 32. In certain embodiments, the solution conduit 30 may not include a conduit valve 32, but rather the flow of the solution may be controlled by the receptacle fitting 28 or other suitable fluid control device or means within the sprayer 22.

The pump 31 draws liquid from a reservoir 29 and delivers it to a plurality of spray nozzles 24, which are part of the sprayer 22. Certain embodiments of the present disclosure may not include the reservoir 29. Rather, the pump 31 may draw the liquid directly from the container 12. However, in the illustrated embodiment, the pump 31 may be a positive displacement pump that is operable to draw fluid from the reservoir 29 and deliver the fluid to one or more spray nozzles 24. The pump 31 may be a piston or syringe pump including a linear actuator in communication with the controller 20. In an alternate embodiment, the system 10 may not include a pump or may include devices to create fluid flow in addition to the pump 31. For example, liquid may be drawn from the container 12 or the reservoir 29 and delivered to the nozzles 24 via a Venturi effect created by the nozzles 24 themselves. As another example, a gas overpressure system may be employed to directly pressurize the container 12 and/or the reservoir 29. As yet another example, fluid delivery to the nozzles 24 may be accomplished or assisted because the container 12 is a pressurized container.

In addition, the fitting 26 may be a quick-connect fitting such that the contents of the container 12 stay in the container until the container is received by the container bay 14. The act of placing the container 12 in the container bay 14 causes a valve to open when the fitting 26 is properly received by the receptacle 28.

The sprayer includes a gantry 33, which supports one or more spray nozzles 24. The gantry 33 is vertically movable via a motor 35 such that the spray nozzles 24 may pass over the entirety of a person standing in front of the nozzles 24. Alternate embodiments may employ a horizontally moving gantry 33 or a gantry 33 that moves in a pre-defined geometric path. In addition, the gantry 33 or the overall sprayer 22 may support an array of fixed nozzles 24.

In the moving gantry embodiment shown, the gantry motor 35 may be controlled by the controller 20 to only pass over the face or the legs of a person as well. In certain embodiments, the controller 20 receives data from the container tag 16 and from that data determines that the solution is particularly suitable for a full body pass. For example, the solution may be a moisturizing solution that is typically applied to the full body of a person. Upon receiving this data, the controller 20 may direct the motor 35 to move the gantry in a manner to accomplish the full body pass to spray the moisturizing solution.

In addition, the gantry 33 may support one or more operational sensors. For example, the gantry may include a sensor 34, such as an optical sensor, for detecting the height of an individual. In this manner, after determining the height of an individual, the nozzles 24 may be directed to accurately pass over only the full body of an individual, which will avoid spraying solution that is not applied to the individual.

The container tag 16 may employ automatic identification and data capture technology. For example, the container tag 16 may include a printed bar code, which may be optically read by the interrogator 18. A two dimensional or matrix code stores significantly more information than a conventional one dimensional bar code, such as a UPC bar code. Thus, a 2-D code may be coded with data that the controller 20 may use to control operating parameters of the sprayer 22. In other embodiments, the container tag 16 may employ radio frequency identification technology (RFID). Regardless whether the technology is bar code, 2-D code, or RFID, container tag 16 is readable by the interrogator 18. In certain embodiments, such as some RFID tags, the tag 16 may be writable from the interrogator 18 as well. In such embodiments, the container tag 16 may receive information from the controller 20 through the interrogator 18, which may then be coded to the container tag 16. This data may be read by the interrogator 18 or similar automatic identification data and capture reader in connection with a subsequent spray session.

Automatic identification and data capture refers to the methods of automatically identifying objects, collecting data about them, and entering that data directly into a computer system without human involvement. The automatic identification and data capture technology, which may be used in embodiments of the container tag 16 and interrogator 18, include bar codes, 2-D codes, 3-D codes, RFID, magnetic stripes, quick response (QR) code, photo recognition, and optical character recognition. However, preferred embodiments of the container tag 16 and the interrogator 18 employ bar code, 2-D code, and/or RFID technology to automatically provide data regarding the contents of the container 12 to the controller 20. Such data is read by the interrogator 18 and received as input by the controller 20.

According to an embodiment of the present disclosure, the container controlled spray system 10 employs an RFID tag 16 that is fixed to the container 12. The RFID tag employs radio frequency electromagnetic fields to transfer data from the tag 16 attached to the container 12 to automatically provide the controller 20 data pertaining to identification, tracking, and other data associated with the contents of the container 12. Some container tags 16 may not require a battery and instead are powered by the electromagnetic fields used to read them, which in the illustrated embodiment, would be provided by the interrogator 18. Other container tags 16 may include a local power source and may emit radio waves (electromagnetic radiation at radio frequencies) that are received by the interrogator 18.

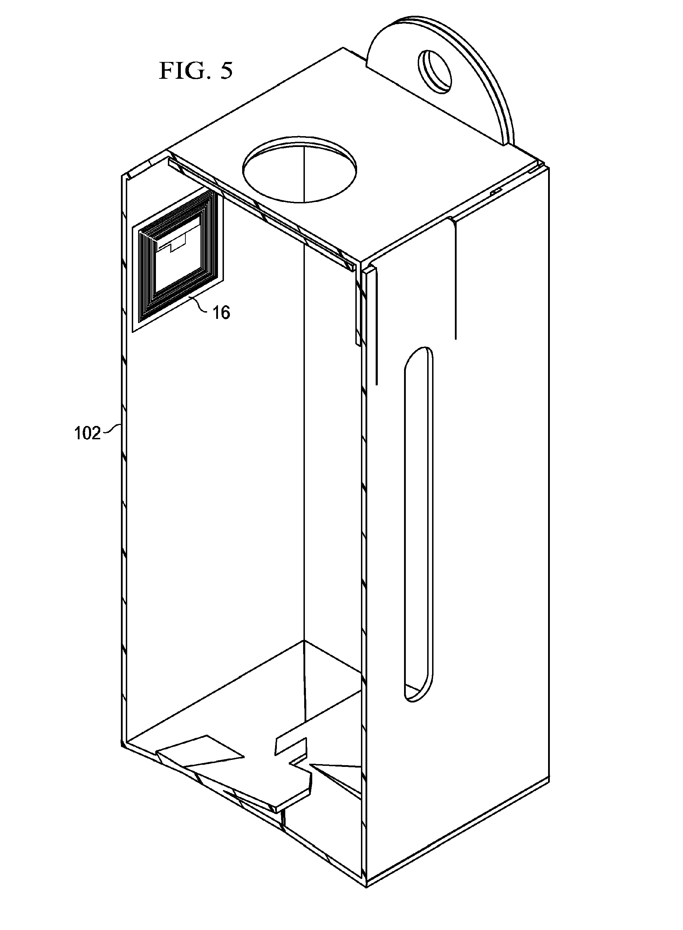

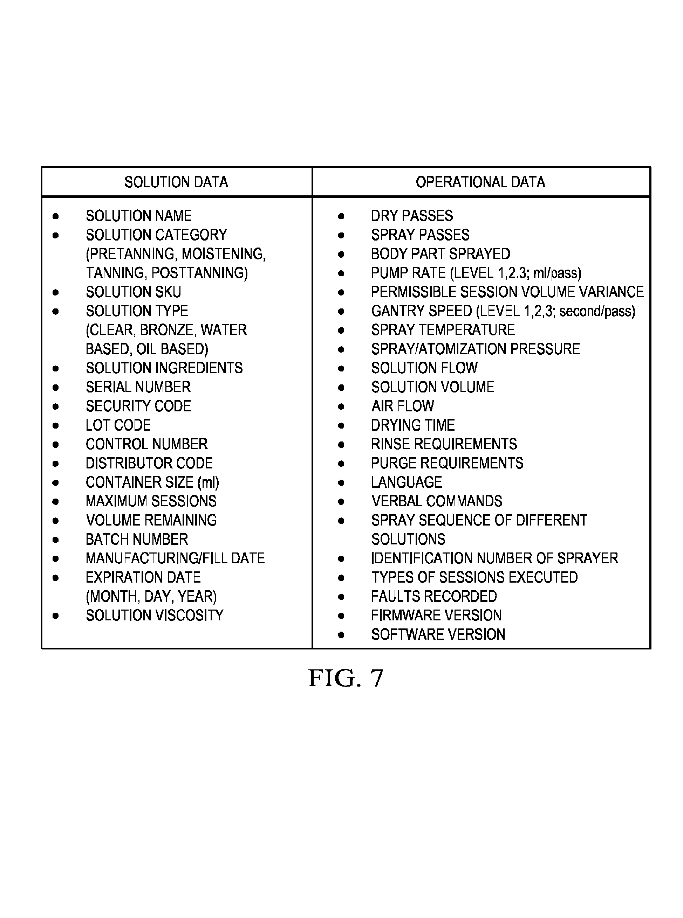

The container tag 16 contains electronically stored information, which may be read from up to several meters away. An RFID container tag 16, unlike a bar code, does not need to be within a line of sight of the reader/interrogator 18, and instead may be embedded in the container 12. Thus, RFID container tag 16 may be secured to the outside of the container 12, it may be embedded within the wall of container 12, it may be printed directly on the container 12 with electrically conductive ink, or it may be adhered to an inside surface of the container 12 (see Figure 5). In certain embodiments, the container 12 may be a bag-in-box container system. In this embodiment, the solution is contained in a plastic bag, which is then contained in box. In this manner, the RFID container tag 16 may be inside the box but still may be separated and protected from the solution because it is outside of the plastic bag. According to an alternate embodiment, data used to configure certain spray parameters of the system 10 is indirectly read or obtained from the container tag 16. In this embodiment, the tag 16 may be a printed bar code or one or more magnetic stripes, or may include characters that can be read and recognized by optical character recognition technology. This tag 16 is interrogated and a code or other identification information is read from the tag 16 and communicated to the controller 20, which in response, accesses a cloud network 56 or other computing device 41 (see Figure 1B). The code includes specific identification information to allow the controller 20 to access a specific portion of the cloud memory 56 associated with that container. For example, the container 12 may include a container tag 16 that has a bar code that is readable by an optical interrogator 18. The bar code is used by the cloud 56 to access storage media in the cloud 56 where a specific file, file folder, database entry etc. is located. The storage media location is identified by the container code. The remote data source may include a stored file, which may be specific to the type of solution in the container 12, or may be specific to a production lot of the solution, or the actual solution in the specific container 12. The stored file may include any of the information shown in Figure 7. For example, the stored file may include detailed information regarding the solution or operational information regarding use of the solution. This information may be retrieved by the controller 20 and used by the sprayer system to perform a variety of functions.

The container tag 16 may be encoded with, or associated with remotely stored, information regarding the contents of the container 12, which may be used to direct the components of a spray session. The initial coding may be done in connection with the filling of the container 12. Figure 7 is a table showing examples of the type of data that may be coded to the container tag 16 or stored remotely and accessible in connection with interrogation of the container tag 16. The coded data may generally be categorized as solution data and operational data. The operational data is primarily the parameters for a spray session. The spray parameters or operational data may include airflow associated with the sprayer, solution flow (pump motor control), drying time, drying intensity, formulation type, rinsing and purging requirements, numbers of spray passes, languages, types and sequences of voice messages to be announced to the user, speed and range of spray gantry movement, and the like. Also, the information associated with the specific solutions in the container 12 may be stored on or otherwise associated with the container tag 16. For example, information such as the specific solution formulation, the fill date, and the volume used also may be stored on the container tag 16 or may be stored remotely and accessed upon interrogation of the container tag 16 according to the teachings of the present disclosure.

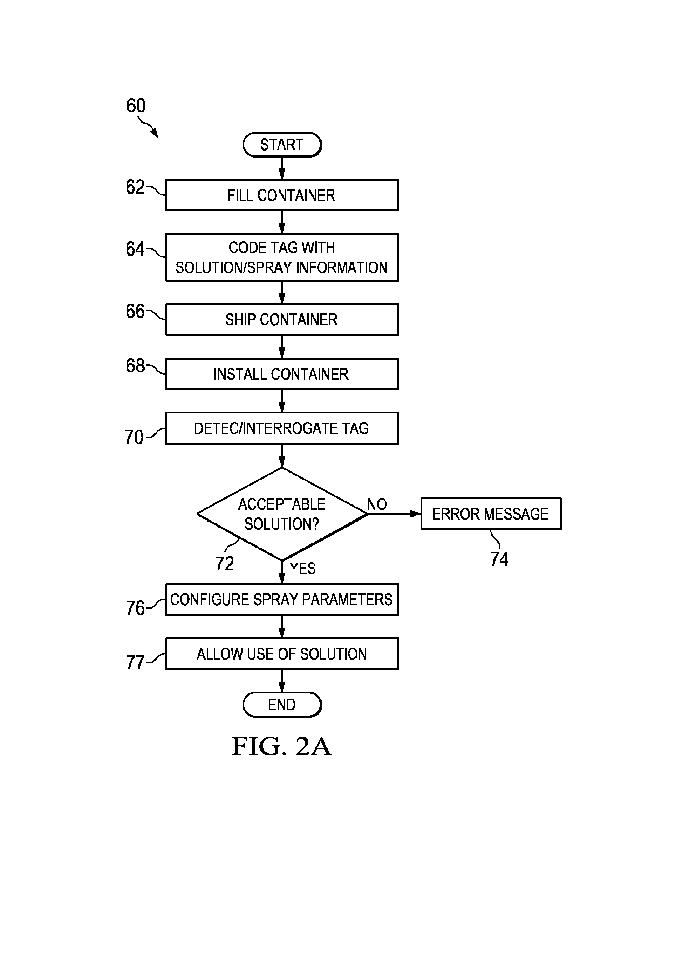

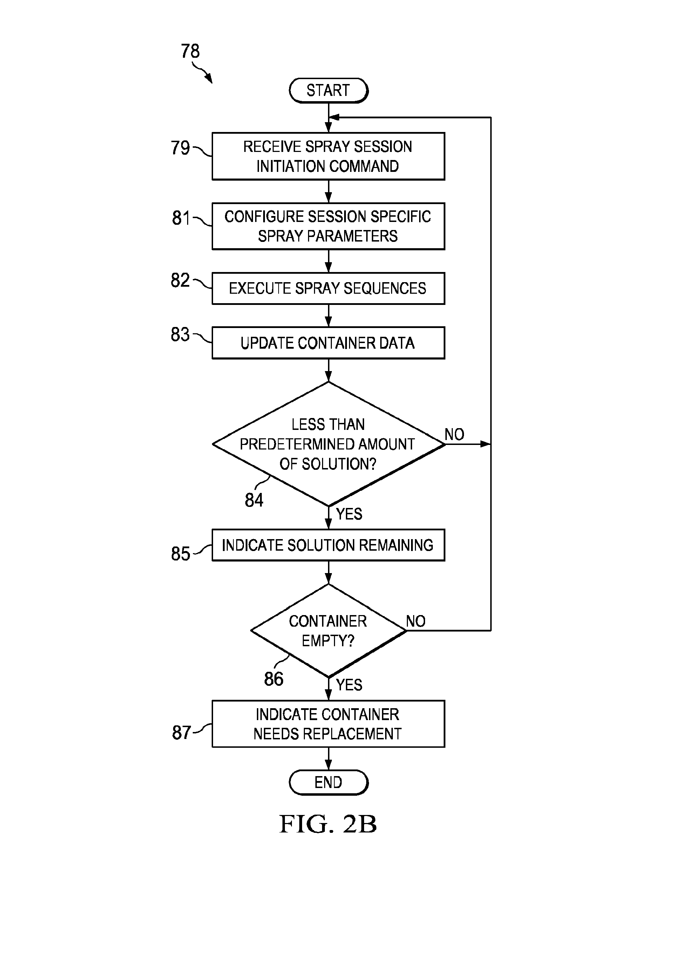

According to certain embodiments, the container tag 16 may function as threshold device to ensure that only containers 12 with solution that is the correct solution and not out of date or expired may be used with the sprayer 22. In this manner, the controller 20 may detect solution specific data from container tag 16, and allow the solution to be sprayed only if certain threshold data matches with expected data as determined by the controller 20. An example of the container tag 16 functioning as a threshold checking device is described with reference to Figure 2.

Reference is now made to Figure 1B, which shows a spray solution data communication system 40. The data communication system 40 includes the controller 20 shown in Figure 1A. Although not illustrated in Figure 1B, the controller maintains its data connections with the pumps 31 and the components associated with the sprayer 22, as shown in Figure 1A.

The controller 20 includes one or more processors 42 and memory 44. Thus, the controller is essentially at least one microprocessor in communication with one or more data storage mediums, such as volatile or non-volatile memory 44, and functions to control the spray session and to communicate data to other elements of the communication system 40. The controller 20 may be remote from the system and may be incorporated into a personal computing device, such as a smartphone, tablet, or laptop computer. For example, the controller 20 communicates with the cloud/network 56, a computing device 41, and an interrogator 18, which reads a code from a container tag 16 on a container 12 of skin treatment solution. The data communication is enabled through WI-FI, Ethernet, Bluetooth, or other suitable data communication protocol.

The controller 20 includes or is otherwise in communication with an interface 46. The interface 46 may be any suitable interface that allows a human to interact with and receive information from the controller 20. In certain embodiments, the interface 46 may be a touch-screen, keypad, monitor, smartphone, tablet computing device, and the like. In a preferred embodiment, the interface 46 may be a touch-screen that allows the user to communicate with the controller 20 by touching the screen where command icons and other information are displayed. The interface 46 and/or the controller 20 may be in communication with speakers 48. The speakers 48 may give audible instructions to the user who is in a spray tanning booth receiving a spray tanning session. The spray session may require the user to take action in response to certain audible commands associated with a particular phase of a spray tanning session.

The controller 20 also may be in communication with one or more operation sensors 50. An operation sensor may be associated with a booth for applying a skin care solution to a human target, such as a tanning booth and the like. The operation sensor may provide the controller 20 with data about the present state of the spraying session. For example, the operation sensors 50 may sense temperature, airflow, solution flow, and the like. The controller 20 may receive this information and the processor 42 may adjust certain spray parameters based on this data. In certain embodiments, the operation sensors 50 may function as safety sensors, thus, if one or more operation sensors 50 detect an unintended condition, the controller 20 may direct the sprayer 22 to shut down and cease the spraying or drying operation.

In certain embodiments, the controller 20 may be in communication with a point of sale (POS) system 52. For example, the controller 20 may use data read directly from or accessed in connection with the interrogation of the container tag 16 such as initial fill volume data to determine a quantity of spray solution used during the spray tanning session. This information may be communicated to the point of sale system 52. In this manner, the user may be charged for a specific quantity and type of spray solution used. Moreover, if multiple spray solutions are used in a spray session, the point of sale system 52 may track the use of each individual spray solution and charge the user accordingly.

The controller 20 may also be in communication with an inventory system 54. The inventory system 54 may possess data regarding the inventory of spray solutions in the inventory of a particular salon or other skin care or spray tanning provider. The controller 20 may communicate information to the inventory system 54 indicating that a certain type of solution has been used and the inventory system 54 may interpret such information as indicating that additional solution of that type should be reordered such that the inventory of the salon or spray tanning operation remains at an appropriate operational capacity. In certain embodiments, the controller may communicate with a solution supplier through a network 56 and reorder the solution automatically.

As previously mentioned, a communication network or cloud 56 and/or computing device 41 allows the controller 20 to communicate data to, and store data at, remote locations. The cloud 56 includes at least one computer and likely a network of computers remote to the rest of the system 40, which may be maintained by a third party in the business of providing cloud computing services. The network of computers generally forms a back-end of the cloud 56 and performs the data storage and processor intense processing functions. Cloud computing is known in the art to allow centralized storage and computing power that can be accessed by computing devices separate from the cloud 56 to take advantage of centralized remote storage and computing power.

Moreover, the cloud network 56 may allow third-parties access to the information obtained by the controller 20 to determine any number of actions that should occur based on such information. In addition, the controller 20 may collect information and distribute it to the computing device 41 or other computers in the cloud/network 56 where this information may be tabulated and further analyzed. Thus, usage information for a particular spray system 10 and/or collection of spray systems may be displayed and further analyzed. Through this data, the operational cycles of components of the spray system 10, such as electrical valves, fans, compressors, drive motors, and the like may be tracked and reported.

From the cloud 56 and/or computing device 41, the controller 20 may receive efficient updates and modifications to spray parameters. For example, it may be determined that optimal performance of a specific type of bronzer solution is achieved if more of the solution is applied during a treatment session. This information may be communicated to the controller 20 from the cloud 56 or computing device 41 and the spray parameters may be adjusted accordingly. Thus, when the container with that type of bronzer solution is installed in the container bay and its container tag 16 is interrogated, this information regarding the type of bronzer may be communicated to the cloud 56 and the current spray parameters may be communicated back to the controller 20. In this manner, the system may maintain current optimal parameters.

In addition to the network 56, the controller 20 may also be in communication with one or more computing devices 41. The computing device 41 may be a laptop computer, a desktop computer, a tablet computer, a smart phone, and the like. The computing device 41 may be associated with an individual or business entity that operates one or more of the container controlled spray systems 10, as shown in Figure 1A. The computing device 41 may include a software application, such as an Internet browser or a dashboard program that allows communication with the back-end computer network of the cloud 56. Thus, the computer program running on the computing device 41 is operable to direct the transmission and receipt of data to and from the cloud 56 and to direct operations of the back-end computer network. The computing device 41 is in communication with the sprayer system 10 through the controller 20.

Reference is now made to Figure 2A, which illustrates a method 60 for using data obtained directly or indirectly from a container tag to configure parameters of a spray system. The spray system includes the elements of container controlled spray system 10 illustrated in Figure 1A. The method begins at step 62 where a container is filled with solution. The container may be filled with a skin treatment solution, such as a spray tanning solution and the container may be a bag-in-box type, disposable, reusable, or other suitable container. At step 64, a machine readable, for example bar code or RFID container tag, is coded with data associated with the skin treatment solution.

According to the embodiment employing data indirectly obtained from the container tag 16, the container tag 16 is encoded with a code or other data that identifies a corresponding remote media storage space. The storage space may be specific to the contents of the particular container 12. For example, the container tag may be coded with solution identifier, such as a serial number or other appropriate identifier. The identifier may be associated with this particular filled container or the collection of containers produced in the fill run. This coded container tag is associated with remote storage media, which is or will be populated with solution data. Populating the data may include manually entering data in a database or it may include receiving data from outside sources.

The container tag may be fixed to the container before or after the tag is coded. The data associated with the coded tag may include any of the data types listed in Figure 7, including solution data and operational data. For example, the data may include an indication of a type of solution, a serial number, and a specific solution volume to be dispensed per spray session. The data may also include specific details regarding the spray sequence that should be employed to apply the particular solution, including an air atomization pressure associated with the fluid qualities that affect flowability and/or atomization, such as the viscosity of the skin treatment solution in the container. The air atomization pressure assures that the solution will be emitted from the nozzle as a finely atomized spray (mist) that provides even coverage and reduces the risk of streaking, speckling, blotches, or running of the spray deposit. In certain embodiments, the coded tag may include a serial number or other identifier and the corresponding spray parameters associated with that identifier may be retrieved from an array stored on the controller or may be retrieved from a remote computing device, which may be associated with a cloud or local computing network.

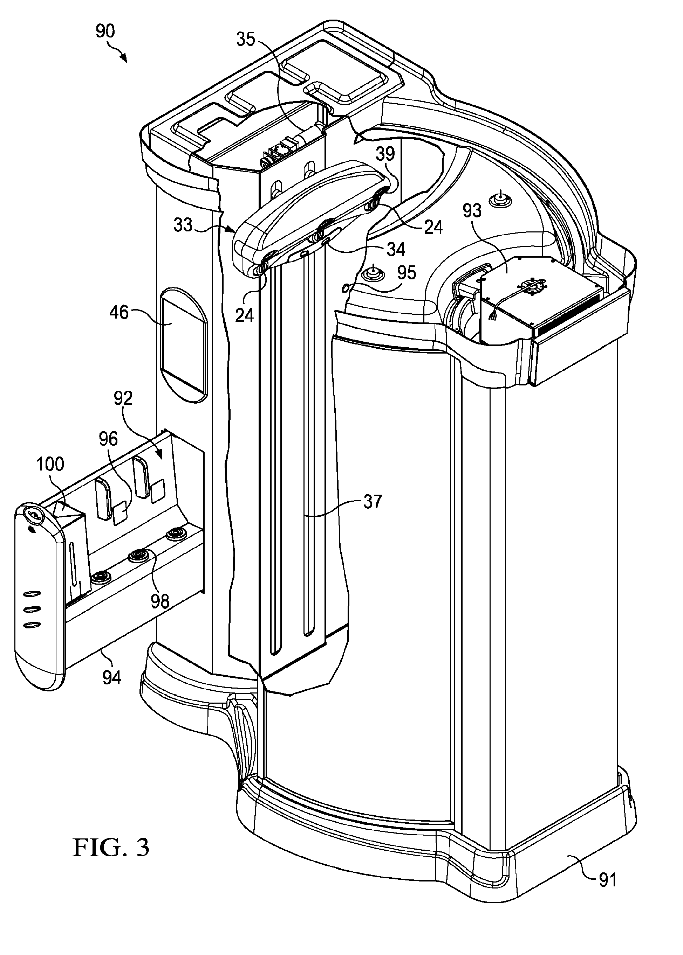

At step 66, the filled container with the container tag is shipped to where it may be used. The filled container may be shipped to a customer that operates skin care spray booths, such as a salon that operates a container controlled spray system according to embodiments of the present disclosure. The salon installs the container into a skin treatment spray booth, for example a spray tanning booth, as shown in Figure 3, at step 68. The installation may proceed as previously described with respect to the block diagram of the container controlled spray system 10. Thus, the container may be received by a container bay 14 or other suitable container receptacle, and the container bay may include an interrogator 18 to interrogate and read the data coded on the container tag.

At step 70, the container tag is detected by the interrogator 18 and a correct installation may be indicated to the user. In an alternate embodiment, the interrogator may be remote from the system 10. For example, the interrogator 18 may be associated with a point-of-sale of a single dose container of skin treatment solution. In this embodiment, the container tag 16 may be interrogated by the remote interrogator 18 in connection with a point-of-sale transaction before it is installed in the system 10. In addition, the container tag is interrogated such that the data encoded on the tag is read by the interrogator and that data is communicated to the controller where it may be stored for later use by the controller in a sprayer operation. The controller may also use the data immediately depending on the system settings associated with receiving that particular container with that particular container tag.

According to an alternate embodiment, upon reading the container tag the controller or other computing device accesses the remote storage media and provides identification information read from the container tag. In response to receiving the identification information, the remote storage media locates the specific data associated with that identifier and communicates that data to be received by the controller. The controller may display the data or any appropriate subset of the data through the interface. This is an optional step, and the controller may not display any of the data received from the remote storage media.

At step 72, it may be determined whether the solution is acceptable for the desired spraying operation based on the data read from the tag or the remotely stored data associated with the container tag. The RFID tag may include information that the controller is expected to receive, such as a predetermined alphanumeric code that indicates that the solution in the container is acceptable to use with the skin care solution spray machine in which it is installed. If the solution is determined not to be acceptable, then an error message may be displayed to the operator at step 74. In certain embodiments, the user may be provided with the option of manually overriding the controller and directing the sprayer system to draw from the container even though it was determined not to contain an acceptable solution or otherwise indicated an error upon interrogating the container tag. If the container is determined to be acceptable, then the spray parameters may be configured based on the data obtained from the container tag at step 76.

The data may be solution specific. For example, an operator may install a bronzer tanning solution into one of four container bays of a skin care treatment spray booth, such as a spray tanning booth. After determining that the bronzer solution is authorized to be used in the skin care treatment spray booth, the controller directs that information be stored that indicates that the bronzer solution has been installed in that particular container bay. Thereafter, if a treatment session includes the application of a bronzer, the spray tanning booth will activate a pump that draws solution from the container in that particular container bay. Solution specific data also includes lot codes, expiration date, and the like.

In addition, the spray tanning machine may determine that a purging operation is necessary because the replacement solution is different from a depleted solution. Thus, the controller may direct the system to initiate pumping cycles to clear the solution conduit of the previous solution. In addition, the machine readable tag may communicate data pertaining to a specific calibration setting that should be used for the specific solution at certain spray treatment levels that may be selected by the user. In response, the sprayer system may automatically perform certain pumping operations associated with that calibration.

At step 77, the system allows the use of the solution drawn from the container associated with the interrogated tag in subsequent spray sessions.

A method 78 for using the data either directly or indirectly obtained from the container tag to control a spray session is illustrated in Figure 2B. At step 79, a command to initiate a spray session is received by the controller. A user may use a button, touch-pad or other interface to provide information indicating that one or more desired spray treatments or levels (quantity of a spray solution) is to occur during a spray session.

Per step 70 of Figure 2A, data previously read from the container tag or obtained from remote storage media associated with the container tag is stored in memory associated with the controller. Alternatively or additionally, the container tag may be interrogated to obtain additional data specific to the solution. Thus, the controller has received data indicating the specific type of solution that is installed in a particular container bay from stored data read in connection with the installation of the container and/or a subsequent container tag interrogation in connection with the spray session. The controller has also received data indicating the spray treatments desired by the user. The system uses this data to configure session specific spray parameters at step 81. Some examples of session specific spray parameters include the number of spray passes for a specific treatment, signals to direct the gantry motor to move the gantry to deliver the spray over the desired part of the body a predetermined number of times or at a certain speed, a solution pump rate indicting a quantity of solution to be delivered to the nozzles per spray pass depending on the selected level and type of solution, and the like.

At step 82, the specific spray sequences associated with the configured spray parameters are executed by the spray skin treatment machine. For example, the controller may direct that a certain number of drying passes and spraying passes in a particular sequence are to be performed to apply the particular solution. The volume of solution delivered in a spray pass may be determined by the container tag associated with the particular solution that is to be delivered. In addition, a specific air atomization pressure may be determined based on the data read from the container tag. For example, a bronzer may have a higher viscosity, and therefore a higher air atomization pressure is employed to create the finely atomized coating mist emitted from the nozzle. The controller sends signals to operate the gantry, atomization air, auxiliary air heaters, and the pumps accordingly.

In one embodiment, the read data corresponds to an adjustment of air pressure of an air compressor system. The air pressure and air flow is adjusted such that it will atomize the skin treatment solution into a mist that will coat the consumer without streaking, speckling, blotching, and the like. In embodiments that include other means to atomize a skin treatment solution, the data may be read and the atomizing means, such as hydraulic pressure, may be automatically adjusted to deliver an appropriate atomized mist based on this data.

Once the skin treatment spray machine has completed the spray sequences, if the container tag is an RFID tag to which information may be written, then the spray tanning system may write information regarding the completed spray sequence to the RFID tag, at step 83. Alternatively, the controller may write information regarding the completed spray sequence to the remote storage media associated with the container tag. For example, a volume amount and a session count that has been decremented from an initial amount or count may be written to the container tag. In addition, a serial number of the skin care treatment spray machine may be written to the container tag. This updated information may be read in connection with a subsequent spray session. In this manner, the container data may be updated after each spray session such that the current information may be saved on the container tag with regard to the specific uses of that spray container. This ability to update the container tag, or remote storage media associated with the container tag, facilitates movement of a particular container among several spray tanning booths. At step 84, it may be determined whether there is less than a predetermined amount of solution remaining in the container. This determination may be made automatically. The RFID tag was initially coded or otherwise associated with data indicating the quantity of the solution in the container, and the spray tanning machine detects the amount of spray tanning solution dispensed from the particular container and thereby may automatically determine the quantity of solution remaining in that container. Thus, at step 85, it may be indicated that the level of solution remaining is less than a predetermined amount. The amount may be a percentage of the original quantity in the container, such as 5%. The amount remaining may also be correlated to a number of spray passes that the container may deliver before the container in empty. Thus, an operator may be warned that the solution will need replacing soon, but the system may continue to be operated for a predetermined number of spray passes before the container is empty.

If there is not less than a predetermined amount of solution remaining, the method returns to step 79 and the system is ready to receive a command initiating a subsequent spray session. Another spray session may be executed because it has been determined that the system has enough solution in its containers to perform the session.

At step 86, it is determined whether the container and/or a corresponding reservoir is empty. If the container is not empty, then the method continues to step 79 where the system may receive a command to initiate a subsequent spray session, even though it has already been determined that the solution remaining is below a certain predetermined threshold amount at previous step 84. If the container is empty, then it may be indicated that the particular container needs to be replaced at step 87 and the method ends.

It should be understood that the reference to a spray tanning machine is an example embodiment. The teachings of the present disclosure are applicable to any sprayer system adapted to spray solutions or other treatments to the human body. Such solutions or treatments may include, but are not limited to, topical medicinal treatments, skin lotions, and the like.

Some of the steps illustrated in Figures 2A and 2B may be combined, modified, or deleted where appropriate, and additional steps may also be added to the flow diagrams. Additionally, steps may be performed in any suitable order without departing from the teachings of the present disclosure.

Reference is made to Figure 3 which shows an isometric view of a tanning booth 90 with portions broken away to reveal internal components. Specifically, a vertically movable gantry 33 supports the plurality of nozzles 24, which in the illustrated embodiment includes three spray nozzles 24. The spray nozzles 24 emit atomization air and liquid skin treatment solution in the form of a finely atomized mist to coat a target skin surface. In addition, the gantry 33 supports an auxiliary heated air outlet 39 above each nozzle 24. The heated air outlets 39 may be used in drying passes and/or may be used to heat the spray cloud emitted by the nozzles 24. A motor 35 moves the gantry 33 vertically along the tracks 37. The gantry 30 also supports the optical height sensor 34.

The spray booth 90 includes an enclosure supported by a base 91. An exhaust fan 93 operates to exhaust overspray out of the enclosure. The spray booth 90 also includes a touch-screen interface 46, which may be part of the controller 20, and the other components described with reference to Figures 1A and 1B. A start sensor 95 is internal to the spray booth 90 and allows a user to initiate a preconfigured spray session once the person has entered the enclosure and is in position to receive the spray from the nozzles 24. The start sensor 95 may be a button, switch, microphone, and the like. In the illustrated embodiment, the start sensor 95 is touch sensitive capacitance switch that communicates a signal to the controller upon being touched by the user.

Also internal to the spray booth 90, but not explicitly illustrated, are a heater, a drying system, lights, a speaker and other components known in the art to be used in connection with the spraying application of skin treatment solutions, such as a spray tanning solution. These components of the spray booth 90 may have their respective parameters configured using the data coded on a machine readable container tag as described herein. Thus, the spray booth 90 may receive information from a bar code or RFID tag and execute conduit purging, spraying, drying, exhaust, and the like sequences to provide a user with a spray tan. According to embodiments of the present disclosure, the spray solution container may be received in a container bay 92. The spray booth 90 also includes a retractable receiver drawer 94 that includes a plurality of container bays 92. In certain embodiments, a receiver drawer may include four container bays 92. Each container bay 92 may be sized and configured to receive a bag-in-box solution container 100. In addition, each container bay 92 includes an interrogator 96. The interrogator 96 functions as described with respect to Figures 1A and 1B. That is, the interrogator 96 reads and may write to the machine readable container tag fixed to, or otherwise associated with, the container received in the corresponding container bay 92. Each container bay 92 also includes a fitting receptacle 98 that receives the fitting of the bag-in-box container of solution and may activate a springloaded valve that allows access to the contents of the bag-in-box container.

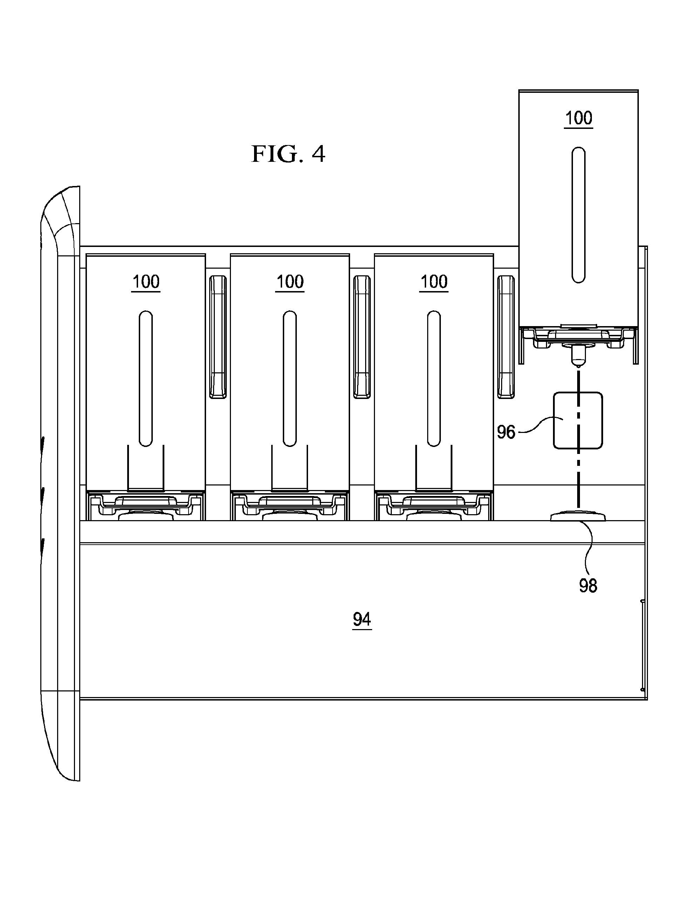

Figure 4 shows a detail of the drawer 94 showing three bag-in-box containers 100 positioned within the drawer 94. A fourth container 100 is shown exploded above a corresponding position in the drawer 94. Each position in the drawer 94 includes an interrogator 96. The interrogators 96 are shown as generally square pads. According to embodiments of the present disclosure, the interrogators 96 may be any size or shape suitable for reading data from a machine readable container tag, for example an RFID tag located on the solution container. The interrogator 96 receives data communicated from the machine readable tag attached to the removable container 100.

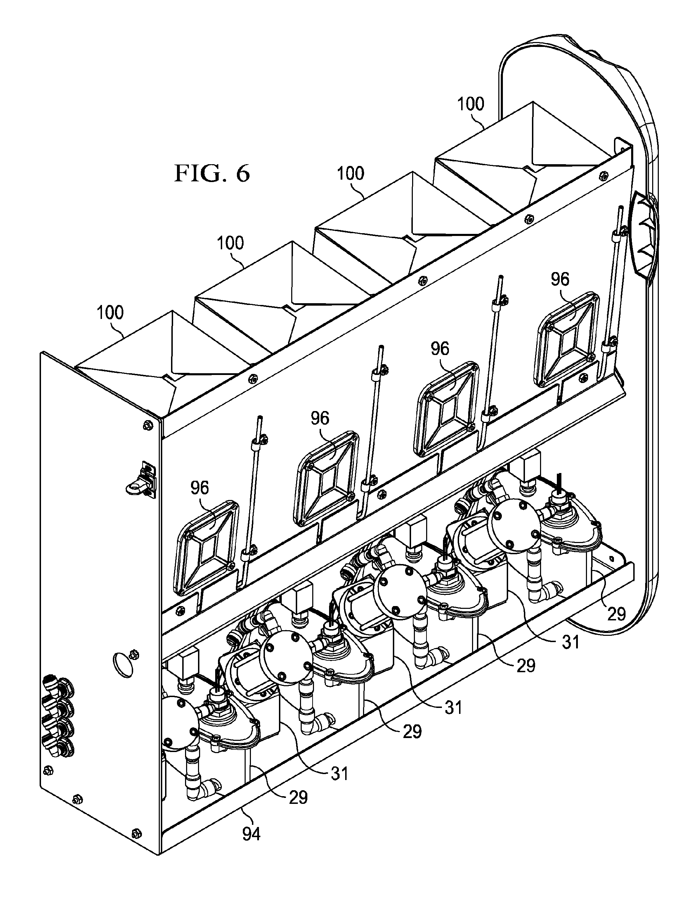

For example, as shown in Figure 5, a radio frequency identification (RFID) tag 16 is attached to an inside surface of a wall of a box 102. A bag and a wall of the box have been removed in Figure 6 to more clearly show the position of the tag 16. The placement location shown is not to be limiting, but rather the placement of the container tag may be any suitable location where it may be read by a corresponding interrogator 96. The solution container 100 may be reusable and may be installed and removed from the container bays 92. The solution container 100 may contain any type of solution used in the application of a cosmetic to the skin in a spraying operation. For example, the solution may be a solution for pre-tanning, moistening, tanning, and post-tanning. The solution may be clear or bronze and may be water or oil based.

According to an alternate embodiment, the container of solution may be received by a receptacle located in a nozzle housing. United States Patent No. 7,992,517 to Cooper, which is hereby incorporated by reference, discloses a gantry tower spraying system with a cartridge receptacle assembly. In this embodiment, the cartridge or solution container includes a machine-readable tag and an interrogator may be associated with the receptacle such that when the cartridge is inserted the machine readable tag may be read, and certain data is obtained from the machine readable tag. The data may be operational data and/or solution data. The data is passed to the controller and certain spray parameters may be configured and executed implemented based on this data.

Figure 6 illustrates a specific embodiment of a portion of a container controlled sprayer system according to the teachings of the present disclosure. Figure 6 shows four pump systems 31, each of which are in fluid communication with a respective reservoir 29.

In addition, the pumps 31 are each in communication with a respective interrogator 96 through the controller. The interrogator may read information stored on a machine readable tag, such as an RFID tag, attached to a removable container 100 of skin treatment solution, such as a sunless tanning solution. Each of the pump systems 31 may be plumbed to allow solution to be pumped from a respective reservoir 29 to the plurality of spray nozzles 24 (see Figure 3). In this manner, each pump 31 may be operable to pump a different solution to the spray nozzles 24. Thus, an individual may receive a spray tanning session that includes treatment from a plurality of different skin treatment solutions. For example, the individual may receive a pre-tanning solution in a first treatment operation from a first reservoir 29 pumped by a first pump system 31. The same person, in a second subsequent treatment operation, may receive a bronzer skin treatment solution pumped by a second pump system 31 and sprayed on the individual. After each tanning session is completed, the remaining volume in the container is written to the container tag, such as an RFID tag, which can be read by the interrogator 96 upon initiation of a subsequent session.

## Claims

1. A skin treatment sprayer system, comprising:
at least one container receptacle configured to receive a container of skin treatment solution;
an interrogator coupled to the at least one container receptacle, the interrogator operable to communicate with a machine readable tag associated with the container;
a controller comprising a processor in communication with the interrogator;
at least one spray nozzle in fluid communication with the container and being operable to emit a spray comprising the skin treatment solution; and
wherein the controller is operable to control a spray parameter associated with the emission of the skin treatment solution based on data received from the machine readable tag.

2. The system of claim 1, wherein the machine readable tag comprises a bar code and the interrogator is operable to read the bar code; or
wherein the machine readable tag is a radio frequency identification (RFID) tag and the interrogator is operable to receive the data from the RFID tag; or
wherein the machine readable tag is selected from a group consisting of: an optically readable tag, a magnetically readable tag, a mechanically readable tag, a photo recognizable tag, and an electronically readable tag.

3. The system of claim 2, wherein the machine readable tag comprises a two dimensional bar code and the interrogator is operable to read the two dimensional bar code; or
wherein the interrogator is operable to write data to the RFID tag.

4. The system according to any one of the preceding claims, further comprising a pump in fluid communication with the container and the at least one nozzle.

5. The system of claim 4, wherein the spray parameter is a pump rate associated with the pump.

6. The system according to any one of the preceding claims, further comprising a movable gantry supporting the at least one spray nozzle; and/or
further comprising an interface in communication with the controller.

7. The system according to any one of the preceding claims, wherein the at least one container receptacle comprises a plurality of container bays, each having a respective interrogator and each being in fluid communication with the at least one spray nozzle.

8. A method for delivering a skin treatment solution, comprising:
receiving a container by a skin treatment spray system, the container containing a skin treatment solution and having a machine readable tag coupled to the container;
receiving data from the machine readable tag; and
controlling delivery of the skin treatment solution by the skin treatment spray system based on the received data.

9. The method of claim 8 wherein the received data comprises solution data and further comprising determining to deliver the skin treatment solution based on the solution data; or
wherein the data comprises operational data and further comprising delivering the skin treatment solution at a predetermined rate based on the operational data.

10. The method of claim 9 wherein the solution data identifies a type of the skin treatment solution.

11. The method of claim 9 wherein the skin treatment sprayer comprises a pump and further comprising operating the pump to deliver the skin treatment solution at the predetermined rate.

12. The method according to any one of the claims 8-11 further comprising determining to purge at least one conduit of the skin treatment spray system based on the received data; preferably wherein the data comprises a value associated with a type of the skin treatment solution and further comprising:
comparing the value to stored value associated with a previous name of a previous skin treatment solution; and
determining to purge the at least one conduit based on the comparison.

13. The method according to any one of the claims 8-12 further comprising determining to calibrate a pump associated with the delivery of the skin treatment solution based on the received data; preferably wherein the data comprises a value identifying the skin treatment solution and further comprising:
comparing the value to a stored value; and
determining to calibrate the pump based on the comparison.

14. The method according to any one of the claims 8-13 wherein the machine readable tag is an RFID tag and the data is a solution volume of the skin treatment solution contained in the container and further comprising writing a value associated with a remaining volume to the machine readable tag after delivering the skin treatment solution; preferably further comprising reading the value associated with the remaining volume before delivering the skin treatment solution in a subsequent spray session.

15. A system for controlling the delivery of a skin treatment solution for a skin treatment spray system according to any of claims 1-7, comprising:
an interrogator operable to interrogate a machine readable tag coupled to a removable container of skin treatment solution;
a controller in data communication with the interrogator, the controller operable to control delivery of the skin treatment solution based at least partially on data obtained either directly or indirectly or both directly and indirectly from the interrogation of the machine readable tag; and
at least one spray nozzle operable to emit a spray including the skin treatment solution.

16. The system of claim 15 wherein the data is obtained indirectly from the interrogation of the machine readable tag and the data is received by the controller from remote storage media; and/or wherein the interrogator is remote from and in wireless data communication with the controller.
